Europäisches Patentamt

European Patent Office

Office européen des brevets

(11) Numéro de publication: **0 224 439**
**A1**

## (12) DEMANDE DE BREVET EUROPEEN

(21) Numéro de dépôt: **86810478.7**

(22) Date de dépôt: **27.10.86**

(51) Int. Cl.⁴: **G 01 N 33/546**
C 12 Q 1/00, B 01 D 15/02

(30) Priorité: **31.10.85 CH 4694/85**

(43) Date de publication de la demande:
**03.06.87 Bulletin 87/23**

(84) Etats contractants désignés:
**CH DE FR GB IT LI SE**

(71) Demandeur: **BATTELLE MEMORIAL INSTITUTE**
**7 route de Drize**
**CH-1227 Carouge/Genève (CH)**

(72) Inventeur: **Place, John Francis**
**42, Chemin de la Chevillarde**
**CH-1208 Genève (CH)**

(74) Mandataire: **Dousse, Blasco et al**
**7, route de Drize**
**CH-1227 Carouge/Genève (CH)**

(54) **Support à phase solide d'un composant destiné à participer à une réaction dans une phase liquide.**

(57) Ce support est formé de granules (5) dont le volume et, par conséquent, la densité sont variables en fonction de la pression et de la température, les densités de ces granules, à deux pressions données, étant respectivement supérieure et inférieure à la densité de la phase liquide (2,3), pour permettre, d'une part, de mélanger les granules dans la phase liquide par l'application de pressions variant entre ces pressions données (fig 1b) et, d'autre part, de séparer ces granules de la phase liquide en appliquant l'une desdites pressions données (fig 1c). Ce support trouve des applications dans la réalisation de tests immunologiques aussi bien que dans des réacteurs bio-chimiques ou chimiques.

FIG. Ia   FIG. Ib   FIG. Ic   FIG. Id   FIG. Ie   FIG. If

EP 0 224 439 A1

**Description**

# SUPPORT A PHASE SOLIDE D'UN COMPOSANT DESTINE A PARTICIPER A UNE REACTION DANS UNE PHASE LIQUIDE

La présente invention se rapporte à un support à phase solide d'un composant destiné à participer à une réaction dans une phase liquide.

Les granules à phase solide trouvent actuellement de plus en plus d'applications dans des réactions en phase liquide, aussi bien à des fins d'analyses (par exemple des tests immunologiques à phase solide) que dans des processus de fabrication (par exemple des systèmes à enzymes ou cellules immobilisées pour la fermentation industrielle).

Dans la plupart des tests immunologiques, un marqueur est utilisé pour mesurer la distribution de complexes antigènes-anticorps et d'antigènes ou d'anticorps seuls dans la réaction à liaison réversible antigène-anticorps, la distribution entre des complexes liés et les parties libres étant alors proportionnelle à la concentration initiale d'antigènes-anticorps avant la réaction.

A moins que le marqueur du test immunologique ne soit modifié par la réaction de liaison (comme dans les tests homogènes, par exemple EMIT® Syva, U.S.A.) la détermination est facilitée par la séparation des complexes liés des parties libres du test (cas du test hétérogène). Ce résultat est souvent obtenu en fixant un composant du test à une phase solide qui est séparée physiquement de la phase liquide après la réaction d'incubation. Plusieurs variantes de tests hétérogènes sont connus et décrits dans la littérature.

Plusieurs types différents de phases solides destinées à des tests de liaisons hétérogènes sont disponibles commercialement. La gamme disponible va des systèmes à un seul support tels que des tubes ou des billes revêtus jusqu'à de la cellulose microcrystalline ou de la silice à granulométrie fine. Le choix de la phase solide pour un test donné est dicté par plusieurs facteurs, notamment l'aptitude à être associée à un agent de liaison (en relation avec la superficie de surface totale disponible) et la commodité d'utilisation.

L'inconvénient de telles phases solides provient du fait qu'elles doivent être dispersées dans la phase liquide pour permettre une réaction complète (à l'équilibre) avec le ou les réactants en solution, et qu'elles doivent ensuite être séparées de la phase aqueuse avant de mesurer le résultat de la réaction.

Donc le support de phase solide pour les tests immunologiques doit satisfaire deux exigences contradictoires. Pour obtenir une dispersion optimale de la phase solide conduisant à une réaction efficace et donc une bonne sensibilité du test, il est nécessaire que la phase solide présente une surface aussi grande que possible dans un volume donné, ce qui peut être obtenu avec les granules fines. Par contre, pour la séparation de la phase solide, plus les granules sont grandes, meilleure elle est. Les solutions adoptées cherchent généralement à réaliser un compromis entre ces deux exigences contradictoires et à trouver des méthodes de mélange et de séparation aussi efficaces que possible.

Pour le mélange par exemple on a proposé de faire osciller des tubes de réaction autour d'un axe transversal médian aux tubes respectifs, le mélange par vortex intermittent, l'agitation intermittente, l'utilisation de granules fines. Pour la séparation, on a eu recours à la centrifugation et au lavage répété de la phase solide (avec mélange intermédiaire), à un gradient de saccharose, à des particules ferro-magnétiques et à des billes de plastique etc..

L'utilisation de particules ferro-magnétiques permet de les séparer facilement du liquide à l'aide d'un champ magnétique. Bien que ces particules soient relativement lourdes, elles restent assez bien en suspension dans la phase liquide à condition d'être très finement divisées. Cependant, de telles particules sont difficiles à remettre en suspension dans la phase liquide.

Avec n'importe lequel des supports à phase solide sous forme de particules ou granules susmentionnés destinés à être mis en suspension dans la phase liquide, il est nécessaire d'avoir recours à deux techniques différentes pour mélanger et séparer ces particules ou granules. Souvent, dans une installation d'analyse automatique, ces techniques ne sont pas évidentes à mettre en oeuvre. C'est ainsi que le mélange, qui doit se faire sans risque de contamination et sans prélèvement de produit, doit de préférence, éviter tout contact avec le liquide à analyser. Les techniques d'agitation, surtout sous de très faibles volumes de liquides, ne sont pas toujours efficaces et reproductibles, en raison des tensions de surface vis-à-vis des parois du récipient notamment. La séparation par centrifugation nécessite généralement le transfert du tube d'analyse sur un rotor de centrifugation, ce qui complique l'installation ainsi que le processus d'analyse et augmente à la fois les coûts et la manipulation.

Une phase solide de dimension intermédiaire réalise un compromis entre les deux extrêmes, c'est-à-dire, les particules finement divisées et un support unique. Une telle phase solide de dimension intermédiaire présente une capacité adéquate d'agent de liaison et si sa densité est sensiblement égale à celle de la phase liquide, alors elle peut rester en suspension pendant la phase d'incubation de la réaction de base.

La demande de brevet EP-A-0123403 se rapporte à des particules de dimension intermédiaire qui se séparent lentement par gravité. Le désavantage d'une séparation pendant l'incubation est évité en augmentant sensiblement la densité de la phase liquide pendant l'incubation (par exemple par addition de saccharose). Cependant cette solution présente certains désavantages. Par exemple l'adjonction d'un agent destiné à modifier la densité peut affecter la réaction de base par des effets chimiques ou physiques sur les propriétés de liaison. L'adjonction d'un tel agent et sa dilution dans la phase liquide peut affecter l'équilibre réalisé et nécessiter des étapes supplémentaires pour réaliser le test.

0 224 439

Des phases solides sont utilisées également de plus en plus dans d'autres domaines que celui de l'analyse immunologique, tels que dans des réacteurs biologiques et biochimiques. Certaines cellules et micro-organismes peuvent être cultivés plus efficacement sur des surfaces appropriées tels que des micro-supports de 50-300 µm, par exemple le Cytodex® de Pharmacia (Suède) utilisés pour la production de virus. d'interféron et d'anticorps. Des virus et des vaccins sont ainsi produits dans des réacteurs de 100-1000 litres en utilisant des particules de support à phase solide.

Etant donné que les micro-organismes croissent et peuvent réaliser plusieurs réactions secondaiers indésirables, il est parfois préférable de n'utiliser que les systèmes enzymes nécessaires à la réaction en masse recherchée. Ces enzymes. étant solubles dans la phase liquide. seraient lavées dans un réacteur continu. C'est la raison pour laquelle elles sont souvent immobilisées sur des phases solides. Quand bien même les organismes ou cellules entiers sont plus stables, qu'ils peuvent synthétiser leurs propres enzymes et arranger le positionnement stérique des réactifs dans les réactions requises, des enzymes sont souvent utilisées pour des raisons pratiques de contrôle et de pureté du produit.

La culture de cellules de mammifères est souvent le seul moyen approprié pour produire des protéines de mammifères, étant donné que les cellules de mammifères réalisent plusieurs des modifications poste-translationnelles nécessaires pour produire des molécules fonctionnelles. La culture de cellules de mammifères n'est pas facile en raison de la fragilité des cellules sensibles aux méthodes de mélange classiques (brassage, agita tion). La croissance des cellules de mammifères étant relativement lente, il est important de maintenir des conditions aseptiques et d'éviter l'accumulation de produits microbiens qui peuvent réduire la croissance des cellules.

Les cellules de mammifères sont cultivées selon deux méthodes - en suspension homogène libre (avec de bonnes conditions de culture) et sous forme immobilisée avec perfusion par la phase liquide (par exemple, dans des réacteurs à fibres creuses, des lits fluidisés ou sur des matrices spongieuses ou en céramique.

Les dispositifs de mélange utilisés actuellement comportent des mécanismes pour remuer, agiter ou faire tourner le récipient de réaction ou pour faire passer des bulles d'air à travers le milieu de réaction. Ces procédés de mélange présentent des désavantages. Le brassage du liquide produit une dislocation par cisaillement et le joint de l'arbre de l'organe de brassage constitue une source de contamination du réacteur. L'agitation et la rotation du réacteur ne sont pas très efficaces et produisent une contamination des parois du réacteur. Le brassage avec des bulles d'air peut provoquer la formation de mousse et la contamination des parois du réacteur.

On utilise de plus en plus les réacteurs à perfusion pour produire à grande échelle des substances à haute valeur ajoutée tels que des anticorps monoclonaux. L'inconvénient des réacteurs à fibres creuses réside dans la chute de pression liée à la perfusion à travers les fibres creuses et à la possibilité de former des poches de cellules isolées avec un micro-environnement. De ce fait, certains producteurs préfèrent encore utiliser des réacteurs à suspension sous forme d'un lit fluidié.

Comme dans les tests immunologiques, les principaux problèmes liés à l'utilisation des phases solides sont relatifs au mélange des phases solides avec les réactifs, pour garantir la réaction complète et la séparation ultérieure des produits de réaction en phase liquide. Dans la production à grande échelle, le mélange est également important pour le transfert de la chaleur de réaction et la distribution de gaz dans la phase liquide.

Le but de la présente invention est de remédier, au moins en partie, aux inconvénients susmentionnés, liés à la nature des supports à phase solide proposés jusqu'ici.

A cet effet, cette invention a pour objet un support à phase solide d'un composant destiné à participer à une réaction dans une phase liquide, selon la revendication 1.

Un avantage essentiel de ce support réside dans le fait qu'un même moyen (c'est-à-dire essentiellement les variations de pression) sert à mélanger aussi bien qu'à séparer le support à phase solide de la phase liquide. Le mélange est obtenu sans venir en contact avec le liquide et sans soumettre le récipient contenant ce liquide à des accélérations et décélérations répétées pour le mélanger. Les variations alternatives de pression permettent de déplacer les supports à phase solide sur toute la hauteur de la phase liquide, quelle que soit la viscosité du liquide, les tensions superficielles du liquide contre la paroi du récipient. La séparation des supports à phase solide de la phase liquide peut être obtenue en créant une pression qui confère aux supports, soit une densité supérieure à celle du liquide, soit une densité inférieure. Comme on peut le constater, de tels supports sont utilisables dans chacune des applications susmentionnées, aussi bien dans des processus d'analyse que dans des réacteurs chimiques ou biologiques, les avantages conférés étant chaque fois ceux de l'efficacité du mélange et de la séparation de la phase solide ainsi que de la simplicité des moyens mis en oeuvre.

Les supports à densité variables, objet de l'invention, sont obtenus par inclusion de gaz sous forme d'une ou plusieurs cellules dans une phase solide en un matériaux déformable élastiquement, selon la technique des matières plastiques expansées. qui se différencient des structures spongieuses par le fait que leurs cellules sont à pores fermés. Cette technique de formation de matière plastique expansée est par exemple décrite dans un article de G.R. Thomas paru dans "Biotech Plastics" de septembre 1965 (pages 552-558) sous le titre "The formation of cellular plastics". L'exemple que l'on décrira ci-après n'est donc pas limitatif et on pourra au contraire avoir recours à toutes les techniques connues d'inclusion de gaz par voie chimique à l'aide d'agents gonflants. par voie physique par vaporisation, par détente à basse pression ou par voie mécanique par inclusion de gaz ou de micro-sphères creuses. Il est donc possible de se reporter à l'abondante littérature relative à ce sujet pour la réalisation de supports à phase solide dont la densité peut varier en fonction de la

3

pression.

Le dessin annexé illustre schématiquement et à titre d'exemple la mise en oeuvre de tests immunologiques utilisant deux variantes du support à phase solide objet de l'invention.

Les figs 1 à 1f se rapportent aux différentes phases d'un test immunologique utilisant la première variante de support.

Les figs 2a à 2f se rapportent aux différentes phases du même test immunologique utilisant la seconde variante de ce support.

Nous allons plus spécialement décrire ci-après un tel support associé à un anticorps en vue de former un support à phase solide destiné à constituer la phase solide dans le cadre d'un test immunologique.

Dans cet exemple. on forme une solution à 4% d'alginate (Protanal® LF 20/60 de Protan A/S, Drammen, Norvège) dans de l'eau distillée chauffée à 80°C pendant 10 minutes et en refroidissant le liquide visqueux résultant à la température ambiante.

On incorpore ensuite à cette solution une très faible quantité de détergent (0,01% de Tween® 20 de (Merck-Schuchardt, Munich) en faisant mousser la solution avec une hélice entraînée à 4000 t/mn et on distribue cette solution goutte à goutte à l'aide d'une pipette, dont la pointe est à 40 cm au-dessus du niveau d'une solution de 0,1M $CaCl_2$ avec remuage constant à l'aide d'un barreau magnétique tournant à 250 t/mn.

Les granules ainsi obtenus ont environ 1 mm de diamètre et renferment des cellules de gaz. Elles sont ensuite lavées dans plusieurs bains d'une solution de 0,1 M $CaCl_2$.

Pour préparer un anticorps à phase solide en vue d'un test immunologique à marqueur enzymatique pour tester l'immuno gamma globuline (IgG) humaine, on prend 25 ml de granules, préparées selon l'exemple ci-dessus, en suspension dans la solution de 0,1 M $CaCl_2$ et on les transfert progressivement par étapes successives dans de l'acétone par des lavages intermédiaires acétone-eau dont la concentration en acétone passe de 10:90, 50:50, 80:20 à 100:0 aux différentes étapes.

Les 25 ml de granules sont alors en suspension dans l'acétone, le volume total étant de 50 ml. On ajoute alors 3 g de carbonyl-diimidazole (Sigma Chem. Co) qui constitue un agent de pontage de la protéine (anticorps) à fixer sur la granule. Le mélange est alors agité pendant 3 heures à 20°C par un mouvement oscillant de 180° d'amplitude autour d'un axe transversal médian. L'excès d'agent de pontage est ensuite éliminé par un lavage répété des granules avec de l'acétone. Les granules ainsi activées sont ensuite équilibrées à l'aide d'un tampon de test aqueux formé de 0,05 mol/l de Barbitone® qui est un acide diéthylbarbiturique de Fluka, Buchs (Suisse) ajusté à pH 8,0 avec de l'hydroxide de sodium (Merk, Darmstadt) et contenant 0,005 M $CaCl_2$, en augmentant progressivement par pas, la concentration de cette solution tampon par rapport à l'acétone: 10:90, 20:80, 100:0.

A la suite de cette opération d'équilibrage, un anticorps est immédia tement ponté aux granules activées. On ajoute aux 25 ml de granules, une solution de protéines (anticorps) en quantité suffisante pour obtenir un volume final, avec le tampon de Barbitone, de 50 ml. La protéine utilisée est une fraction enrichie d'immuno gamma globuline anti-humaine de mouton (Scottish Antibody Production, Carluke, Ecosse) qui réagit avec les granules à une concentration finale de 5 mg de protéine/ml de volume total. Après une agitation par mouvement oscillant de 180° d'amplitude de l'éprouvette autour d'un axe transversal médian pendant 16 heures. les granules sont séparées de la phase liquide. Pour enlever les substances adhérant aux granules de manière non covalente, ces granules sont ensuite soumises à des lavages répétés à des niveaux de pH alternatifs de pH 2,0 à pH 8,0. La concentration mesurée de protéines liées aux granules était d'environ 1µg/cm².

Les granules préparées selon le processus qui vient d'être décrit et liées aux anticorps, dont la taille est de l'ordre du millimètre, sont alors prêtes pour être utilisées en vue d'effectuer un test immunologique sur un échantillon de sérum humain à analyser. Le processus de réalisation de ce test sera décrit par la suite.

Dans un premier temps on a procédé à une mesure de vérification de la sensibilité des granules à phase solide pontées aux anticorps, obtenues selon le processus décrit ci-dessus. A cet effet on a procédé chaque fois à l'analyse de deux tests, avec deux échantillons de granules, réalisés avec des solutions d'antigènes constitués d'IgG normalisées (avec des concentrations allant de 0,1-100 µg/ml) préparée dans le tampon de test (Barbitone ® 0,05 mol/l, pH 8,0) avec 2% v/v de sérum normal de mouton destiné à remplir les sites non occupés par les antigènes. Pour réaliser ces analyses, on a ajouté 100 µl de solution d'antigènes normalisée à 100 µl de tampon de test, suivi de 50 µl de tampon et 50 µl d'anticorps anti-humains marqués par une enzyme (Peroxidase de Dakopatts, Copenhague, Danemark) en dilution finale dans un rapport de 1:500 dans un tampon.

Après incubation pendant 90 mn à 22°C, les granules ont été lavées trois fois avec le tampon de test et deux fois avec le tampon d'enzyme (0,15 mol/l, de tampon acétate pH 5,0). La réaction colorimétrique, pour détecter l'anticorps spécifique marqué immobilisé, a été réalisée en ajoutant 300 µl de solution chromogène (20 mg/l orthophenanthraline diamine), 20 µl d'une solution de 50% $H_2O_2$ 50 ml de tampon acétate (0,1 mol/l, pH 5,0) et mise à incuber à 22°C pendant 30 mn. La réaction colorimétrique a été stoppée et stabilisée en ajoutant 1 ml de 0,1N $H_2SO_4$. Le taux d'ab sorption a été mesuré à 450 nm par rapport au 0,1N $H_2SO_4$ seul.

Le tableau ci-après montre. pour chacun des deux échantillons, la relation en fonction de la dose obtenue avec le test immunologique à marquage enzymatique pour l'IgG humain, à l'aide des granules selon l'invention.

# T A B L E A U

| Concentration d'IgG humain | Echantillon 1 à 450 nm | Echantillon 2 à 450 nm |
|---|---|---|
| 0 | 0.105 | 0.098 |
| 0.1 | 0.101 | 0.107 |
| 0.5 | 0.220 | 0.190 |
| 1.0 | 0.311 | 0.290 |
| 5.0 | 0.589 | 0.575 |
| 10.0 | 0.701 | 0.705 |
| 50.0 | 0.795 | 0.789 |
| 100.0 | 0.895 | 0.926 |

La sensibilité obtenue peut être estimée à environ 0,5 μg IgG/ml.

On va maintenant décrire à titre d'exemple deux mises en oeuvre, pour réaliser des tests immunologiques, impliquant l'utilisation de deux variantes de granules à densité variable comme supports à phase solide d'anticorps d'IgG anti-humains.

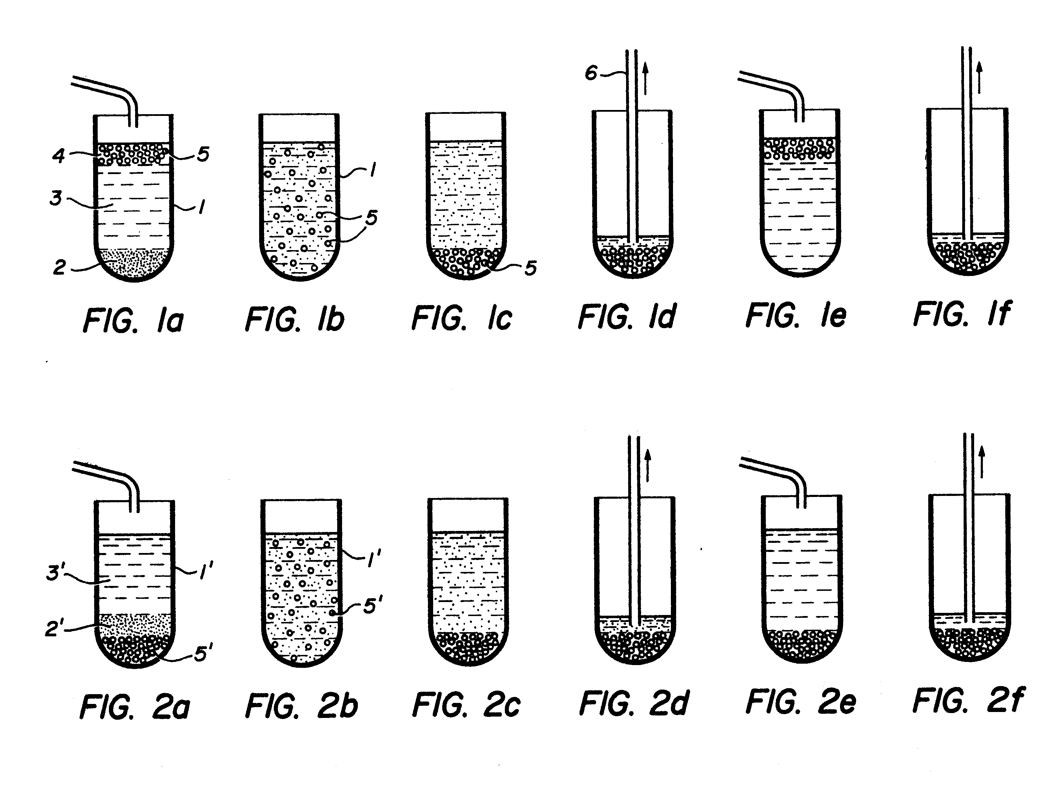

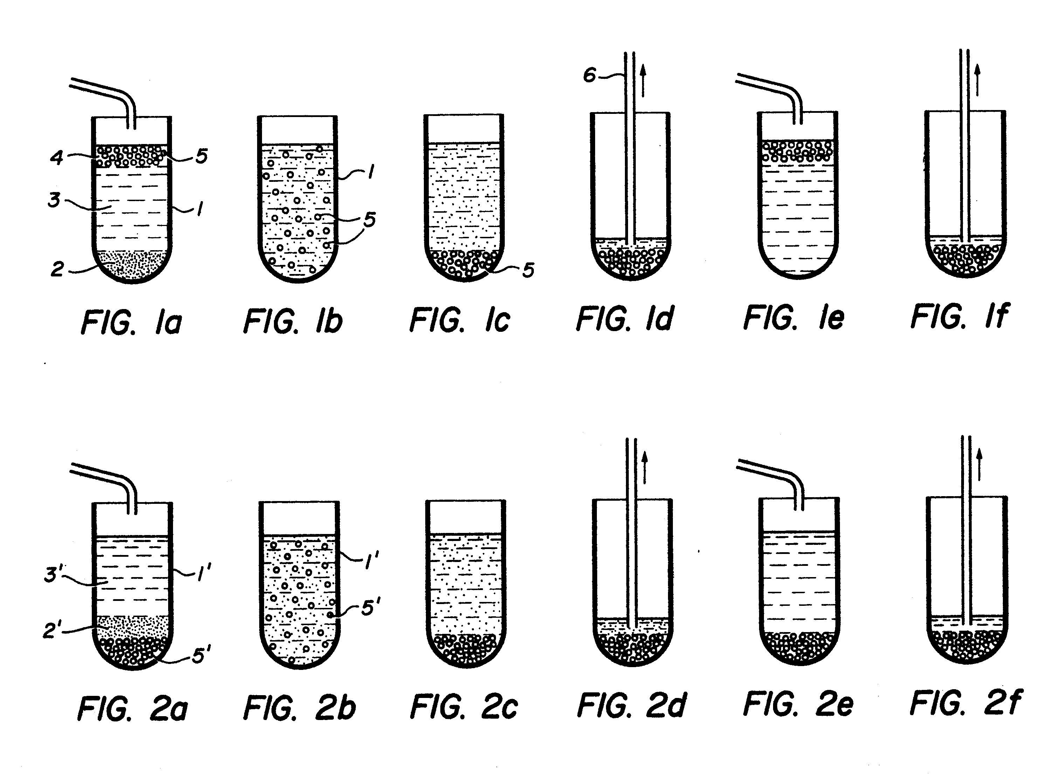

On se reportera tout d'abord aux figures 1a à 1f pour la description de la première mise en oeuvre. Dans un premier temps, (fig 1a), on introduit dans un tube d'essai 1, un volume déterminé d'échantillon à analyser 2 ou d'une solution normalisée utilisée en parallèle (lorsqu'on désire effectuer un test comparatif) avec une quantité déterminée d'un marqueur enzymatique. On ajoute un volume déterminé de tampon 3 et une dose 4 de granules 5 constituées de supports à phase solide d'anticorps selon l'invention. Dans cet exemple, la densité de ces granules est inférieure à celle du liliquide, de sorte qu'à la pression atmosphérique et à la température ambiante, les granules restent à la surface du liquide.

Dans la deuxième phase du procédé, on soumet alternativement le con tenu du tube d'essai 1 à des pressions qui varient entre la pression atmosphérique et une pression supérieure, choisie pour que le volume des granules 5 diminue suffisamment pour leur conférer une densité supérieure à celle du liquide, afin que ces granules descendent vers le fond du tube d'essai 1, puis remontent en fonction des variations de pression. Une fois les granules en suspension (fig 1b) on peut maintenir une pression intermédiaire ou la faire varier en permanence. Pendant ce temps, qui correspond à l'incubation, le liquide est maintenu à une température déterminée pendant une durée donnée.

Une fois l'incubation terminée, la pression est portée à sa valeur supérieure afin de ramener toutes les granules 5 au fond du tube 1 (fig 1c) et le liquide surnageant est aspiré par un conduit 6 (fig 1d).

Dans l'opération suivante, la pression atmosphérique est rétablie et un tampon de lavage est ajouté aux granules 5, après quoi on répète les opérations des figures 1b à 1d avec ce liquide de lavage. On procède ainsi à plusieurs lavages successifs.

On peut ensuite amorcer la phase de coloration d'une solution comprenant un réactif enzymatique comme décrit précédemment en mélangeant à nouveau les granules dans cette solution comme on l'a déjà fait précédemment (fig. 1b) et en faisant incuber à température contrôlée, après quoi on ajoute la solution susmentionnée pour bloquer et stabiliser la réaction, on augmente la pression à sa valeur supérieure, (fig. 1c), pour réunir à nouveau les granules au fond du tube 1. On peut alors procéder à des mesures classiques, en particulier dans ce cas, à la mesure du taux d'absorption d'un rayonnement électromagnétique par le liquide, à une longueur d'onde donnée, en aspirant le liquide (fig 1f) pour l'amener dans un photomètre (non représenté) ou en mesurant à travers le tube. On peut également faire un comptage Gamma des granules dont l'anticorps à réagi avec un antigène humain, dans le cas d'un marqueur isotopique.

On constate, de la description qui précède, que l'utilisation des granules à densité variable comme support à phase solide d'un composant d'une réaction, ici une réaction immunologique, permet de mélanger et de séparer la phase solide sans agir sur le tube lui-même, mais uniquement sur son contenu et seulement par la pression, donc sans contact avec la phase liquide. Le processus mis en oeuvre pour conduire la réaction, se prête particulièrement bien à une automatisation, seule une source de pression variable étant nécessaire pour la réalisation de tout le processus. Il suffit donc d'isoler le tube de l'atmosphère et de le relier à une source de pression. L'efficacité du mélange aussi bien que celle de la séparation sont d'une reproductibilité et d'une fiabilité totale.

Dans la variante illustrée par les fig 2a à 2f, la seule différence provient du fait que les granules 5' ont une densité à la pression atmosphérique supérieure à celle du liquide, de sorte qu'elles se trouvent normalement au fond du tube d'essai 1'. Dans ce cas, c'est donc une variation alternative de pression négative par rapport à la pression atmosphérique, qu'il faut appliquer au liquide pour faire monter les granules afin de les mélanger ou de les mettre en suspension dans la phase liquide (fig 2b). Lorsqu'on désire séparer les granules pour aspirer le liquide, il suffit de rétablir la pression atmosphérique. Cette variante peut présenter des avantages par rapport à la précédente, dans la mesure où toutes les opérations autres que celle correspondant au mélange ou à la mise en suspension des granules illustré par la fig. 2b se déroulent à la pression atmosphérique, et le maintien du vide dans une enceinte fermée est plus facile à réaliser que le maintien de la pression. Par ailleurs, le processus de test est semblable à celui décrit précédemment.

Selon une autre variante, les granules à phase solide ont pratiquement la même densité à température et pression ambiante que la phase liquide. Dans cette variante, les granules restent en suspension pendant la phase d'incubation correspondant aux figures 1b ou 2b et des variations de pression sont utilisées pour séparer la phase solide ou la remettre en suspension. Cette variante présente l'avantage supplémentaire que l'incubation peut être réalisée hors de tout appareil particulier dans un récipient quelconque.

Comme on l'a déjà mentionné, les granules à phase solide servant de support d'un composant d'une réaction biochimique ou chimique peuvent être utilisées dans d'autres applications que celles des tests immunologiques décrits. Ce tels supports sont en effet utilisables dans d'autres types d'analyses aussi bien que dans des processus de fabrication utilisant des réacteurs en cuve ou des réacteurs continus. Dans de tels réacteurs continus dans lesquels un écoulement de réactifs entre en continu dans le récipient duquel sortent, aussi en continu, les produits contenant la phase aqueuse, les granules à phase solide peuvent être maintenues en suspension dans le milieu de réaction, en ajustant la pression de façon adéquate. Il est possible d'équilibrer la flottabilité négative des granules à l'encontre du mouvement ascendant des réactifs et d'obtenir une sorte de lit fluidisé moins dépendant de la vitesse d'écoulement de la phase liquide.

Dans le cas de la culture de cellule, on sait que la réunion de granules dans un espace réduite augmente la formation d'agrégats de particules et de cellules, augmentant le rendement de la culture. Dans le US-A-4,335,215, on a proposé d'obtenir cet effet par un transfert périodique d'une suspension de micro-supports, dans un tambour séparé de volume réduit. En utilisant les granules à phase solide selon l'invention, il est possible de les concentrer soit en haut, soit au fond d'un réacteur, pour favoriser la formation d'agrégats, sans transférer la suspension du corps principal du réacteur.

On peut également utiliser ces granules à densité variable comme support à phase solide pour la séparation chromatographique de composés organiques ou inorganiques d'une phase liquide (aqueuse), en recouvrant le support à phase solide d'une substance susceptible de séparer le composé en solution de la phase liquide, par exemple une substance adsorbante. Si ces granules sont placées dans un écoulement de la phase liquide, on peut adapter la pression pour les empêcher d'être entraînées par ce courant. Dans le cas d'une séparation discontinue, les granules peuvent être déplacées dans le liquide en faisant varier leur densité comme décrit précédemment.

Il est évident que la force centrifuge pourrait constituer un des moyens aptes à varier la pression hydrostatique appliquée aux granules à densité variable en vue de réduire leur volume et d'augmenter leur densité.

On a décrit jusqu'ici des applications dans lesquelles les granules selon l'invention constituent des supports à phase solide d'un composant destiné à participer à une réaction, ou d'un matériau susceptible de fixer un composant en solution. Il existe cependant de nombreux cas dans lesquels le mélange de deux composants ou plus dans une phase liquide pose des problèmes qui sont mal résolus par les solutions connues. Dans bien de ces cas, l'utilisation de granules à densité variable en fonction de la pression permettrait d'apporter une solution sans pour autant que ces granules, comme jusqu'ici, constituent un support d'un composant d'une réaction. Dans ces cas, de telles granules ne joueraient que le rôle d'organes de mélange. De ce fait, elles pourraient être de plus grande dimension que les granules décrites précédemment, pour déplacer de plus grands volumes de liquide et favoriser ainsi la formation de remous au sein de ce liquide.

**Revendications**

1. Support à phase solide d'au moins un composant destiné à participer à une réaction dans une phase liquide, caractérisé par le fait qu'il se présente sous une forme de granule en un matériau élastiquement compressible, dont le volume est susceptible de varier en fonction de la variation d'au moins un des paramètres physiques, température-pression, appliqués à cette granule, ses densités, à deux volumes respectifs correspondant chacun à des valeurs déterminées desdits paramètres, étant respectivement supérieure et inférieure à celle de ladite phase liquide.

2. Support selon la revendication 1, caractérisé par le fait qu'il est en un matériau polymère expansé.

3. Support selon la revendication 1, caractérisé par le fait qu'à température ambiante et pression atmosphérique, la densité desdites granules est choisie inférieure à celle de ladite phase liquide.

4. Support selon la revendication 1, caractérisé par le fait qu'à température ambiante et pression atmosphérique, la densité desdites granules est choisie égale à celle de ladite phase liquide.

5. Support selon la revendication 1, caractérisé par le fait qu'à température ambiante et pression atmosphérique, la densité desdites granules est choisie supérieure à celle de ladite phase liquide.

6. Support selon la revendication 1, caractérisé par le fait que ledit composant est un antigène ou un anticorps.

7. Support selon la revendication 1, caractérisé par le fait que la grosseur desdites granules est comprise entre 0,1 et 5 mm.

8. Support selon la revendication 1, caractérisé par le fait que ledit composant est une enzyme.

9. Support selon la revendication 1, caractérisé par le fait que ledit composant est une protéine de pontage spécifique.

10. Support selon la revendication 1. caractérisé par le fait que ledit composant est de l'acide nucléique.

11. Support selon la revendication 1, caractérisé par le fait que ledit composant est une cellule ou un organisme vivant.

12. Utilisation dudit support à phase solide selon la revendication 6, pour effectuer un test immunologique, caractérisée par le fait que l'on mélange une quantité déterminée desdits supports présentant la forme de granules dans la phase liquide en choisissant lesdits paramètres à des valeurs situées entre les deux dits paramètres température-pression déter minés pendant la formation de complexes antigène-anticorps liés auxdits supports et on applique ensuite l'un des deux paramètres température-1pression déterminés pour permettre le prélèvement de la phase liquide, séparément desdits supports à phase solide.

13. Utilisation du support à phase solide selon la revendicaton 1, pour séparer un composant contenu dans une phase liquide, caractérisée par le fait que l'on associe auxdites granules un agent de fixation dudit composant, on donne auxdits paramètres température-pression des valeurs variant entre lesdits paramètres déterminés pour mélanger ces granules à la phase liquide, et on applique ensuite l'un des deux paramètres pression-température déterminé pour permettre de séparer lesdites granules de ladite phase liquide après fixation dudit composant.

14. Utilisation dudit support selon la revendication 1, pour séparer un composant contenu dans un écoulement formé à l'aide d'une phase liquide, caractérisée par le fait que l'on associe auxdites granules un agent de fixation dudit composant, et on choisit un paramètre pression-température en fonction du débit et de la viscosité de ladite phase liquide, conférant auxdites granules une densité susceptible de s'opposer à la force d'entraînement engendrée par ladite phase liquide en écoulement, de manière à former un lit fluidisé desdites granules.

15. Procédé de mélange en phase liquide, caractérisé par le fait que l'on ajoute à cette phase liquide au moins un corps en un matériau élastiquement compressible susceptible de varier de volume en fonction d'au moins un paramètre physique pression-température, la densité de ce corps à deux desdits paramètres pression-température de valeur déterminés étant respectivement supérieure et inférieure à celle de ladite phase liquide, et on varie lesdits paramètres entre lesdites valeurs déterminées pour déplacer ce corps de haut en bas et inversément afin de provoquer des remous au sein de ladite phase liquide, consécutivement au déplacement de ce corps.

0224439

FIG. 1a  FIG. 1b  FIG. 1c  FIG. 1d  FIG. 1e  FIG. 1f

FIG. 2a  FIG. 2b  FIG. 2c  FIG. 2d  FIG. 2e  FIG. 2f

0224439

FIG. 1a   FIG. 1b   FIG. 1c   FIG. 1d   FIG. 1e   FIG. 1f

FIG. 2a   FIG. 2b   FIG. 2c   FIG. 2d   FIG. 2e   FIG. 2f

Office européen des brevets

**RAPPORT DE RECHERCHE EUROPEENNE**

Numero de la demande

EP 86 81 0478

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 123 403 (BOOTS-CELLTECH DIAGNOSTICS LTD) <br> * Revendications * <br><br> ----- | 1,5 | G 01 N 33/546 <br> C 12 Q 1/00 <br> B 01 D 15/02 |

DOMAINES TECHNIQUES RECHERCHES (Int. Cl.4)

G 01 N
C 12 Q
B 01 D

Le présent rapport de recherche a été établi pour toutes les revendications

| Lieu de la recherche | Date d'achèvement de la recherche | Examinateur |
|---|---|---|
| LA HAYE | 04-02-1987 | MEYLAERTS H. |